(19) Europäisches Patentamt
European Patent Office
Office européen des brevets

(11) Veröffentlichungsnummer: **0 202 625**
**B1**

# (12) EUROPÄISCHE PATENTSCHRIFT

(45) Veröffentlichungstag der Patentschrift: **01.08.90**

(51) Int. Cl.[5]: **C07D 211/90**

(21) Anmeldenummer: **86106700.7**

(22) Anmeldetag: **16.05.86**

(54) **Verfahren zur Herstellung von 2,6-Dimethyl-4-(3'-nitrophenyl)-1,4-dihydropyridin-3,5-dicarbonsäure-3 beta-(N-benzyl-N-methylamino)-ethylester-5-methylester und dessen Hydrochlorid-Salz.**

(30) Priorität: **21.05.85 YU 847/85**

(43) Veröffentlichungstag der Anmeldung:
**26.11.86 Patentblatt 86/48**

(45) Bekanntmachung des Hinweises auf die Patenterteilung:
**01.08.90 Patentblatt 90/31**

(84) Benannte Vertragsstaaten:
**AT CH DE LI SE**

(56) Entgegenhaltungen:
**DE-A- 2 218 644**
**DE-A- 2 847 237**

**CHEM. PHARM. BULL., Band 27, Nr. 6, 1979, Seiten 1426-1433; M. IWANAMI et al.: "Synthesis of new water-soluble dihydropyridine vasodilators"**
**J. HET. CHEM., Band 12, April 1975, Seiten 363-365; B. LOEV et al.: "Hantzsch-type" dihydropyridines. IV (1). Carboxylic acids"**
**Angew. Chemie, 90 (1978), 556**

(73) Patentinhaber: **LEK, tovarna farmacevtskih in kemicnih izdelkov, n.sol.o, Verovskova 57, 61000 Ljubljana(YU)**

(72) Erfinder: **Antoncic, Ljubo, Dipl.-Ing., Pidmilscakova 43, YU-61000 Ljubljana(YU)**
Erfinder: **Jazbec, Iztok, Brilejeva 12, YU-61000 Ljubljana(YU)**
Erfinder: **Kocjan, Darko, Dr.-Ing., Ul. Narodne zascite 1, YU-61000 Ljubljana(YU)**
Erfinder: **Krivec, Ivana, Zgornje Jarse 9, yu-61235 Radomlje(YU)**

(74) Vertreter: **Patentanwälte Müller-Boré, Deufel, Schön, Hertel, Lewald, Otto, Postfach 26 02 47 Isartorplatz 6, D-8000 München 26(DE)**

**EP 0 202 625 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 2,6-Dimethyl-4-(3'-nitrophenyl)-1,4-dihydropyridin-3, 5-dicarbonsäure-3β-(N-benzyl-N-methylamino)ethylester-5-methylester der Formel

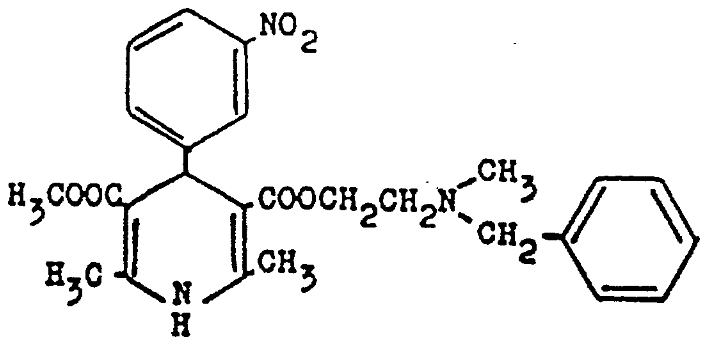

und dessen Hydrochlorid-Salz. Die Verbindung ist unter dem Freinamen Nicardipine (auch YC-93 genannt) bekannt. Nicardipine ist ein cerebraler und koronarer Vasodilatator und wird besonders in der Therapie von cerebraler Insuffizienz verwendet.

Technisches Problem

Es besteht ein ständiges Bedürfnis, ein neues, technologisch leicht durchführbares Verfahren zur Herstellung von Nicardipine zu schaffen, nach welchem die gewünschte Substanz mit einer hohen Gesamtausbeute und Reinheit ohne zeitraubende chromatographische Reinigungen der intermediären Produkte bzw. des Endproduktes und ohne fraktionierte Destillation im Hochvakuum erhältlich wäre.

Stand der Technik

Nicardipine wurde zuerst in DE-PS 2 407 115 bzw. in der äquivalenten US-PS 3 985 758 als eine neue Substanz beschrieben, die besonders als cerebraler Vasodilatator aktiv ist. In dieser Literaturquelle sind mehrere Syntheseverfahren zur Herstellung von Nicardipine, dargestellt im Schema I, angeführt. Ein Nachteil der beschriebenen Verfahren sind geringe Ausbeuten an Nicardipine. Ausserdem müssen, um Nicardipine mit hoher Reinheit herstellen zu können, Nicardipine selbst und die intermediären Verbindungen durch Säulenchromatographie bzw. durch fraktionierte Destillation im Hochvakuum gereinigt werden. Die geringe Ausbeute an Nicardipine ist durch die Herstellung der intermediären Verbindungen, wie 2-(N-Benzyl-N-methylamino)-äthyl-acetoacetat, welches auf eine anspruchsvolle Weise durch Umsetzung von 2-(N-Benzyl-N-methylamino)äthanol mit Aethylacetoacetat in einem wasserfreien Medium in einer sehr geringen Ausbeute von 10,5% hergestellt wird (M. Iwanami et al., Chem. Pharm. Bull. 27(6), 1426-144 (1979)), bedingt. Die Gesamtausbeuten an Nicardipine gemäss DE-PS 2 407 115 betragen zwischen 2 und 12%. Im Beispiel 7 der genannten DE-PS wird der günstigste Weg unter Vermeidung von schwer zugänglichen Zwischenverbindungen beschrieben, wobei Nicardipine in einer Gesamtausbeute von etwa 12% durch Umsetzung von intermedärem 2,6-Dimethyl-4-(3'-nitrophenyl)-1,4-dihydropyridin-3,5-dicarbonsäure-3 -chloräthylester-5-methylester mit N-Methyl-benzylamin erhalten wird.

SCHEMA I

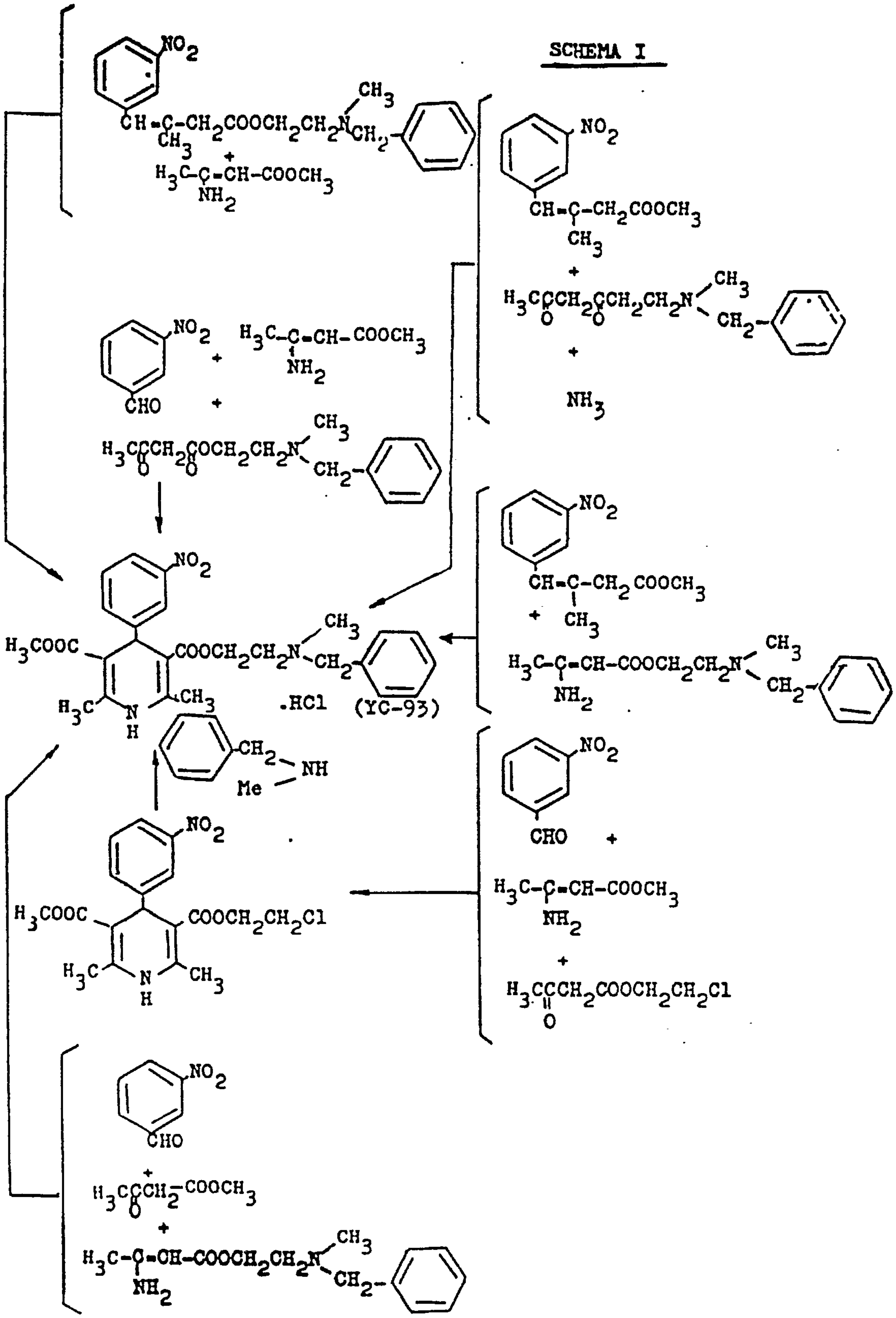

(YC-93)

Es ist bekannt, dass N-Aryl- bzw. N-Alkyl-substituierte Dihydropyridin-3, 5-dicarbonsäure-diester unter Einwirkung von Alkalien zu entsprechenden Dihydropyridin-Monocarbonsäuren hydrolysiert werden (A. Sausins et al., Khim. Geterocikl. Soedin., 2,272 (1978). Im Gegensatz zur leicht durchführbaren alkalischen Hydrolyse von N-Aryl- und N-Alkyl-substituierten Dihydropyridin-3,5-dicarbonsäure-diestern werden N-unsubstituierte Dihydropyridin-3,5-dicarbonsäure-diester überhaupt nicht oder nur in sehr niedrigen Ausbeuten zu entsprechenden Dicarbonsäure-Monoestern hydrolysiert (N. Eisner et al., Chem. Rev. 72, 1, 4 (1972) oder B. Loev et al., J. Heterocyclic Chem. 12, 363 (1975)). Gemäss M. Iwanami et al., Chem. Pharm. Bull. 27(6), 1426-1440 (1979) und T. Shibanuma, Chem. Pharm. Bull., 28(9), 2809-2812 (1980), werden bei partieller Hydrolyse auf die im folgenden Schema II dargestellte Weise etwas günstigere Ergebnisse erzielt.

SCHEMA II

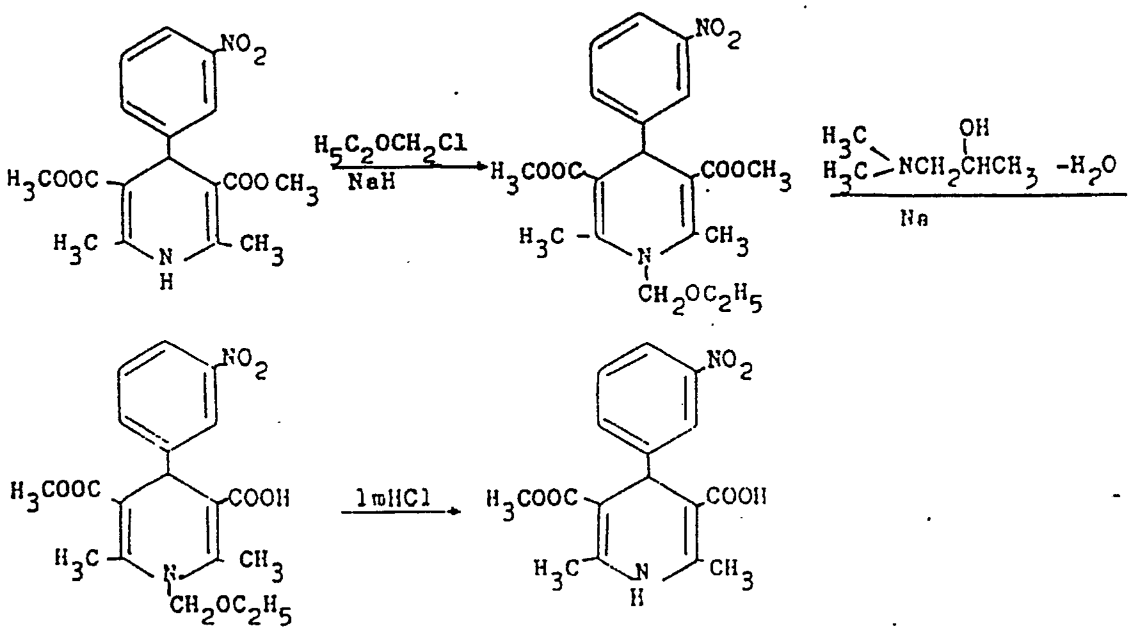


Bei dieser Arbeitsweise wird durch die Einführung der Aethoxymethyl-Grruppe in die 1-Stellung im Kern des Dihydropyridindicarbonsäure-Diesters zuerst 1-Aethoxymethyl-2,6-dimethyl-4-(3'-nitrophenyl)-1,4-dihydropyridin-3,5-dicarbonsäure-dimethylester erhalten, welches dann auf bekannte Weise mit 1-Dimethylamino-2-propanol, welches etwa 2 % Wasser enthält, in einer etwa 43%igen Ausbeute zum entsprechenden Dicarbonsäure-monoester hydrolysiert wird, aus welchem dann durch milde saure Hydrolyse die Aethoxymethylgruppe entfernt werden kann, um 2,6-Dimethyl-4-(3'-nitrophenyl -1, 4-dihydropyridin-3,5-dicarbonsäure-3-methylester zu erhalten. Allerdings ist dieses Verfahren zeitraubend und auch die Ausbeuten sind nicht befriedigend.

Aus dem Artikel von B. Neises und W. Steglich, Angew. Chemie 90 (1978) 556, ist bekannt, daß Carbonsäureester durch die Umsetzung der Carbonsäure mit Alkoholen oder Thiolen in Anwesenheit von 4-Dimethylaminopyridin als Acylierungskatalysator und von Dicyclohexylcarbodiimid in Methylenchlorid hergestellt werden können.

Beschreibung der Lösung des technischen Problems mit Ausführungsbeispielen

Es wurde nun überraschenderweise gefunden, dass man 2,6-Dimethyl-4-(3'-nitrophenyl)-1,4-dihydropyridin-3,5-carbonsäure-3-methylester welches als Schlüssel-Zwischenverbindung in der erfindungsmässigen Nicardipine-Synthese eingesetzt wird, mit hoher Ausbeute durch partielle Hydrolyse von 2,6-Dimethyl-4-(3'-nitrophenyl)-1, 4-dihydropyridin-3,5-dicarbonsäure-dimethylester mit einer wässrigen Lösung von Alkalihydroxiden, wie Lithiumhydroxidhydrat oder Natriumhydroxidhydrat in Anwesenheit von niederen Alkoholen, wie Methanol, als inerten organischen Lösungsmitteln, bei einer Temperatur zwischen Raumtemperatur und Rückflusstemperatur des Reaktionsgemisches erhalten kann. Dabei wird das Alkalihydroxid im Ueberschuss verwendet.

Diese leicht und mit hoher Ausbeute verlaufende partielle Hydrolyse der Ausgangsverbindung, d.h. des N-unsubstituierten Dihydropyridindicarbonsäure-diesters, ist im Vergleich zum Stand der Technik

tatsächlich überraschend und unerwartet, da anhand der bekannten Literatur zu erwarten wäre, dass die partielle Hydrolyse des Ausgangsstoffs 2,6-Dimethyl-4-(3'-nitrophenyl)-1,4-dihydropyridin-3,5-dicarbonsäure-dimethylesters überhaupt nicht stattfinden wird.

Das als Ausgangsstoff in der vorliegenden Synthese von Nicardipine eingesetzte 2,6-Dimethyl-4-(3'-nitrophenyl)-1,4-dihydropyridin-3,5-dicarbonsäure-dimethylester ist eine bekannte Verbindung und kann in hoher ausbeute gemäss der Hantzschen Methode der Synthese von 1,4-Dihydropyridinen durch Umsetzung von 3-Nitrobenzaldehyd, Acetessigsäuremethylester und Ammoniak bei der Rückflusstemperatur des Reaktionsgemisches in Anwesenheit eines inerten organischen Lösungsmittels erhalten werden.

Die andere Schlüssel-Zwischenverbindung in der erfindungsmässigen Synthese von Nicardipine ist N-(2-Hydroxyäthyl)-N-benzyl-methylamin der Formel

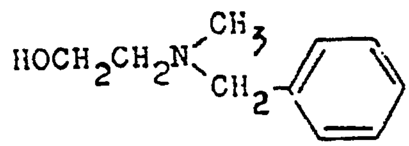

bzw. N-(2 - Halogenäthyl)-N-benzyl-methylamin der Formel

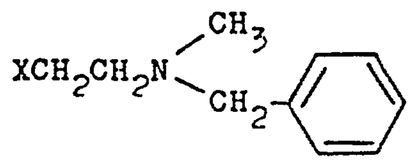

worin X Halogen, wie Chlor oder Brom, bedeutet.

N-(2-Hydroxyäthyl)-N-benzyl-methylamin ist eine aus der Literatur bekannte und beschriebene Verbindung (J.Am.Chem.Soc. 76, 4920-23, 1954). Bei der Umsetzung dieser Verbindung mit Thionylchlorid entsteht in grosser Ausbeute N-(2-Chloräthyl)-N-benzyl-methylamin. Die letztgenannte Verbindung kann auch durch die Umsetzung von N-Benzyl-methylamin mit 1-Brom-2-Chloräthan hergestellt werden, die Ausbeuten sind jedoch gering, und das dimere 1,2-Bis-N-benzylmethylamino)äthan, welches als Nebenprodukt entsteht, muss chromatographisch abgetrennt werden.

Aus DE-OS 2 847 237 ist ein Verfahren zur Herstellung von N-unsubstituierten 1,4-Dihydropyridin-monocarbonsäuren durch alkalische Hydrolyse von 1,4-Dihydropyridin-carbonsäureestern in Anwesenheit von einem inerten Lösungsmittel und Wasser in einem Temperaturbereich von 10 bis 100°C, bekannt. Zur Teilhydrolyse werden als Lösungsmittel niedere aliphatische Alkohole und 1,2-Dimethoxyethan im Temperaturbereich von 20 bis 50°C eingesetzt.

In DE-OS 2 218 644 ist ein Verfahren (Verfahren f) zur Herstellung von basischen 1,4-Dihydropyridin-estern durch Umsetzung einer 1,4-Dihydropyridin-carbonsäure oder deren Alkalimetallsalz mit einer Halogenverbindung in Anwesenheit inerter organischer Lösungsmittel bei Temperaturen zwischen 20 und 150°C beschrieben.

Erfindungsgemäss werden die beiden Schlüssel-Zwischenverbindungen, d.h. 2,6-Dimethyl-4-(3'-nitrophenyl)-1,4-dihydropyridin-3,5-dicarbonsäure-3-methylester der Formel

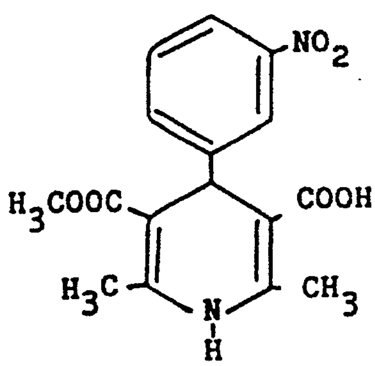

und entweder

a) N-(2-Hydroxyäthyl)-N-benzyl-methylamin der Formel

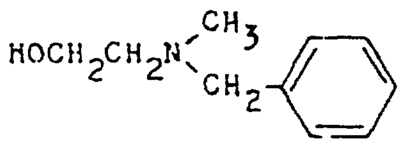

in An- oder Abwesenheit eines organischen Lösungsmittels und in Anwesenheit von N,N'-Dicyclohexylcarbodiimid (DCC) als Kondensationsmittel bei einer Temperatur zwischen 25° und 120°C oder
b) N-(2-Halogenäthyl)- N-benzyl-methylamin der Formel

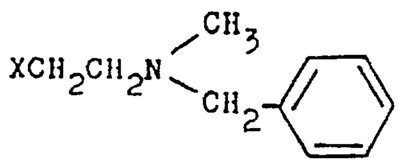

worin X Halogen, wie Chlor oder Brom, bedeutet,
in Anwesenheit von inerten organischen Lösungsmitteln und einer organischen oder anorganischen Base als Protonenakzeptor bei einer Temperatur zwischen 25° und 140°C zu 2,6-Dimethyl-4-)(3'-nitrophenyl)-1,4-dihydropyridin-3,5-dicarbonsäure-3β-(N-benzyl-N-methylamino)äthylester-5-methylester, welches auf an sich bekannte Weise in das entsprechende Hydrochlorid-Salz (Nicardipine-Hydrochlorid) überführt wird, umgesetzt.

Diese Verbindung kann in zwei Kristallformen (α- undβ-Form), die sich durch den Schmelzpunkt, das IR-Spectrum und das Röntgendifraktogramm unterscheiden, isoliert werden, wie das in der oben angegebenen DE-PS bzw. von M. Iwanami et al., Chem. Pharm. Bull. 27(6), 1426-1440 (1979), beschrieben wird.
Als inerte Lösungsmittel werden niedere Alkohole, wie n-Butanol, als Protonenakzeptor eine anorganische oder organische Base. z.B. Triäthylamin o.ä., verwendet.
Die Gesamtausbeute an Nicardipine-Hydrochlorid beträgt bei der Variante a) 46 % und bei der Variante b) 29%, berechnet auf die Ausgangsstoffe.
Das erfindungsmässige Verfahren ist im Schema III dargestellt.
Darin ist ausserdem auch eine Verfahrensvariante mittels Umsetzung des intermediären 2,6-Dimethyl-4-(3'-nitrophenyl)-1,4-dihydropyridin-3, 5-dicarbonsäure-3-methylesters mit 1-Brom-2-chloräthan dargestellt. Bei dieser Variante sind jedoch die Ausbeuten gering und das dimere Nebenprodukt 1,2-Bis(N-benzylamino)äthan muss chromatographisch abgetrennt werden. Das erhaltene 2,6-Dimethyl-4-(3'-nitrophenyl)-1,4-dihydropyridin-3,5-dicarbonsäure-3-chloräthylester-5-methylester kann dann zu Nicadipine bzw. zu seinem Hydrochlorid umgesetzt werden, wie in DE-PS 24 07 115 (Beispiel 7) beschrieben wird.
Die Umsetzungen der Ausgangsverbindungen mit 1-Brom-2-chloräthan, die schlechtere Ergebnisse bringen, werden nur zur Illustration angeführt.

## SCHEMA III

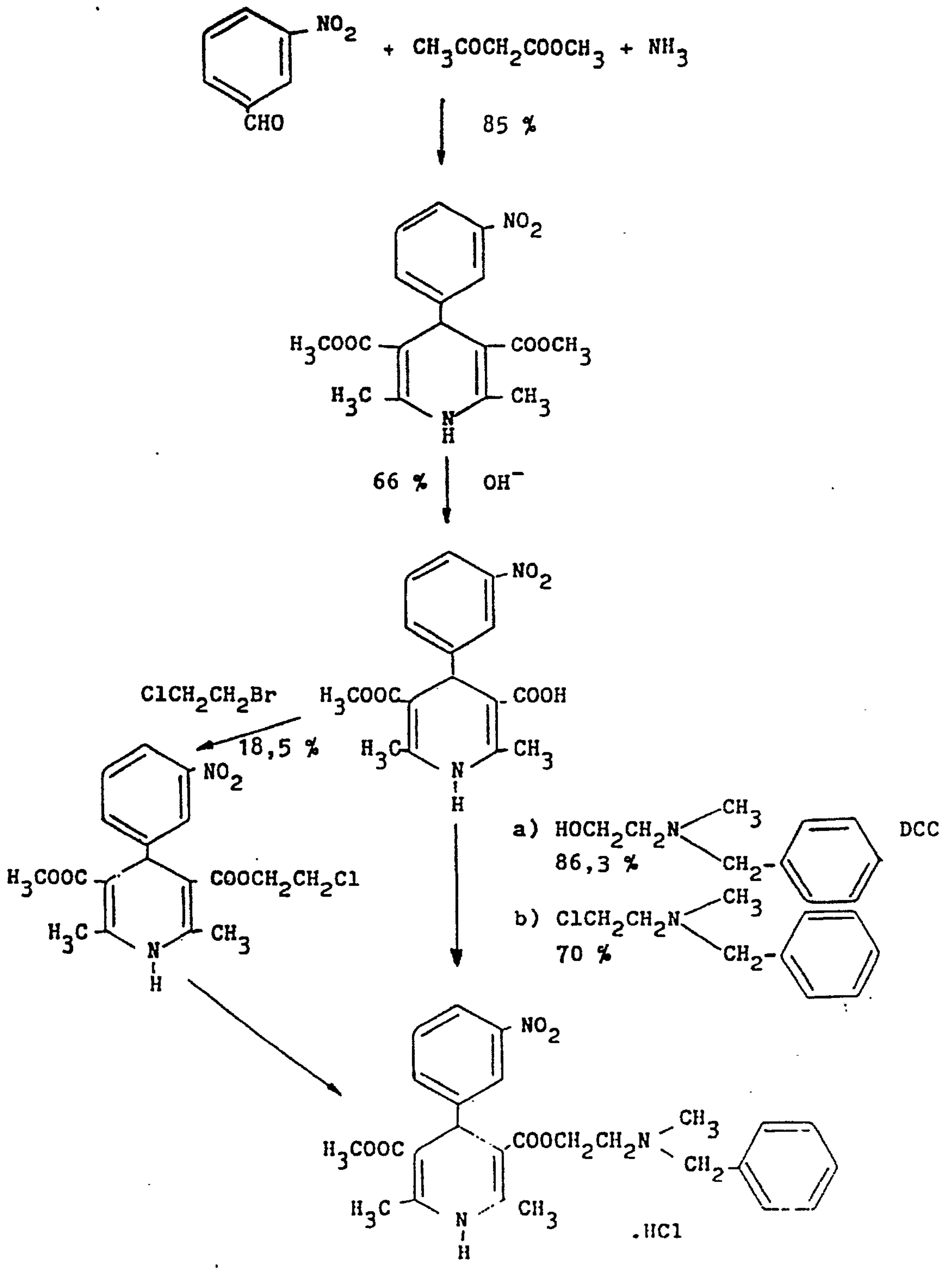


NICARDIPINE (YC-93)

Die Erfindung wird anhand folgender Beispiele, die sie jedoch keineswegs einschränken sollen, illustriert.

Beispiel 1

a) N-(2-Chloräthyl)-N-benzyl-methylamin

I.

Zu einer Lösung von 16,5 g (0,1 Mol) N-(2-Hydroxyäthyl)-N-benzylmethylamin in 100 ml Chloroform werden allmählich 13 g (0, 11 Mol) Thionylchlorid unter Rühren bei Raumtemperatur unter Ausschluss von Feuchtigkeit gegeben. Es wird zur Rückflusstemperatur des Reaktionsgemisches erhitzt und bei dieser Temperatur noch 30 Minuten gerührt. Das Reaktionsgemisch wird dreimal mit je 200 ml einer 10 %igen wässerigen Lösung von $NaHCO_3$ und zweimal mit 200 ml Wasser gewaschen. Die organische Schicht wird mit wasserfreien $Na_2SO_4$ getrocknet, das Trocknungsmittel wird entfernt und das Filtrat wird zur Trockne eingedampft.

Es werden 14,3 g (78 %) der Titelverbindung in der Form eines öligen Rückstandes erhalten.
Bruttoformel: $C_{10}H_{14}NCl$
Molekulargewicht: 183

NMR ($CHCl_3$) $\delta$: 2,4 (3H, s, $>N-CH_3$) 2,8 (2H, t, $>N-\underline{CH_2}CH_2-$)
3,65 (2H, t, $>N-CH_2\underline{CH_2}-$) 3,7 (2H, s, $C_6H_5-\underline{CH_2}-$)

II.

Eine Lösung von 12,1 g (0,1 Mol) N-Benzylmethylamin, 14,3 g (0,1 Mol) 1-Brom-2-chloräthan und 14 g (0,14 Mol) Triäthylamin in 100 ml Toluol wird bei Rückflusstemperatur des Reaktionsgemisches 4 Stunden gerührt. Das organische Lösungsmittel wird abgedampft und der ölige Rückstand wird in einer geringen Menge einer Aethylacetat/Methanol/Ammoniak-Mischung 80:30:3 gelöst und an einer Säule (4 x 35 cm), die mit Kieselgel gefüllt ist, chromatographiert, um die beiden erhaltenen Produkte zu trennen. Es werden 2 g (10,9 %) N-(2-Chloräthyl)-N-benzylmethylamin und 4 g (14,9 %) des dimeren 1,2-Bis(N-benzylmethylamino)äthans als ölige Substanzen erhalten.
Bruttoformel: $C_{10}H_{14}NCl$
Molekulargewicht: 183
NMR ($CDCl_3$) der Titelverbindung:

$\delta$: 2,4 (3H, s, $>N-CH_3$) 2,8 (2H, t, $>N-\underline{CH_2}CH_2-$)
3,65 (2H, t, $>N-CH_2\underline{CH_2}-$) 3,7 (2H, s, $C_6H_5-\underline{CH_2}-$)

NMR ($CDCl_3$) der dimeren Verbindung:

$\delta$: 2,2 (3H, s, $>N-CH_3$) 2,6 (4H, s, $>N-CH_2CH_2-N<$)
3,55 (2H, s, $C_6H_5-\underline{CH_2}$)

b) 2,6-Dimethyl-4-(3'-nitrophenyl)-1,4-dihydropyridin-3,5-dicarbonsäure-3-methylester

17,3 g (0,05) Mol) 2,6-Dimethyl-4-(3'-nitrophenyl)-1,4-dihydropyridin-3, 5-dicarbonsäure-dimethylester werden in 280 ml Methanol suspendiert und unter Rühren bei Raumtemperatur mit einer Lösung von 15,9 g (0,4 Mol) NaOH in 52 ml Wasser versetzt. Es wird bei Rückflusstemperatur 5 Stunden gerührt, das Reaktionsgemisch wird abgekühlt, unter Rühren mit 1050 ml Wasser versetzt und filtriert. Der Niederschlag ist die nicht umgesetzte Ausgangsverbindung, d.h. 2,6-Dimethyl-4-(3'-nitrophenyl)-1,4-dihydropyridin-3, 5-dicarbonsäure-dimethylester. Das klare Filtrat wird mit 1 g Aktivkohle versetzt und bei 50°-60° 30 Minuten gerührt. Es wird abgekühlt, die Aktivkohle wird abgefiltert, das Filtrat wird mit 1 n HCl bis pH 2,5 angesäuert und der ausgeschiedene Niederschlag wird abgefiltert. Der Niederschlag wird noch 2-mal mit je 15 ml Wasser nachgewaschen. Es werden 11 g (66% ) der reinen Titelverbindung, Smp. 202-206°C, erhalten.
Bruttoformel: $C_{16}H_{16}N_2O_6$
Molekulargewicht: 332

NMR ($DMSO-d_6$) $\delta$ : 2,4 (6H, s, $C_{2,6}-CH_3$) 3,6 (3H, s, $-COOCH_3$) 5,1 (1H, s, $C_4-H$) 9 (1H, s, $>N-H$)

c) 2,6-Dimethyl-4-(3'-nitrophenyl)-1,4-dihydropyridin-3,5-dicarbonsäure-3β-(N-benzyl-N-benzyl-N-methylamino)-äthylester-5-methylester-hydrochlorid (Nicardipine-Hydrochlorid)

A) Eine Lösung von 3,32 g (0,01 Mol) 2,6-Dimethyl-4-(3'-nitrophenyl)-1, 4-dihydropyridin-3,5-dicarbonsäure-3-methylester, 1,83 g (0,01 Mol) N-(2-Chloräthyl)-N-benzyl-methylamin und 1,4 g (0,014 Mol) Triäthylamin in 60 ml n-Butanol wird 2 Stunden auf 120°C erhitzt. Das Reaktionsgemisch wird zu einem öligen Rückstand, welcher das rohe 2,6-Dimethyl-4-(3'-nitrophenyl)-1,4-dihydropyridin-3,5-dicarbonsäure-3 -(N-benzyl-N-methylamino)-äthylester-5-methylester (Nicardipine-Base) enthält, eingedampft.

Der erhaltene ölige Rückstand wird in 22,2 ml Chloroform gelöst, mit 16,0 ml einer 10 %igen Salzsäure gewaschen und danach noch dreimal mit je 10 ml Wasser nachgewaschen. Die organische Schicht wird mit wasserfreiem $Na_2SO_4$ getrocknet und das Lösungsmittel wird unter vermindertem Druck abdestilliert. Der ölige Rückstand wird in 17 ml Aethylacetat gelöst und bei 0°C 2 Stunden gerührt. Der ausgeschiedene Niederschlag wird abgefiltert, im Vakuum getrocknet und aus Aceton umkristallisiert. Es werden 3,6 g (70,0 %) des gewünschten Produktes (Nicardipine-Hydrochlorid) welches bei 129- 132°C (unter Zersetzung) schmilzt, erhalten.

Hydrochlorid:
Bruttoformel: $C_{26}H_{30}N_3O_6Cl$
Molekulargewicht: 515
NMR (Base) ($DMSO-d_6$)

$\delta$: 2,21 (3H, s, $-N\langle^{CH_3}_{CH_2-}$ ) 2,36 (6H, s, $C_{2,6}-CH_3$)
2,7 (2H, t, $>N-CH_2-$) 3,5 (2H, s, $-CH_2-C_6H_5$)
3,7 (3H, s, $-COO-CH_3$) 4,2 (2H, t, $-COOCH_2-$)
5,15 (1H, s, $C_4-H$) 9,13 (1H, s, $>N-H$)

B) Ein Gemisch von 3,32 g (0,01 Mol) 2,6-Dimethyl-4-(3'-nitrophenyl) -1, 4-dihydropyridin-3,5-dicarbonsäure-3-methylester, 4,98 g N-(2-Hydroxyäthyl)-N-benzyl-methylamin und 2,05 g N,N'-Dicyclohexylcarbodiimid (DCC) wird bei 60° bis 80°C 1 Stunde gerührt. Das Reaktionsgemisch wird dann mit 16 ml Chloroform versetzt und dreimal mit je 50 ml Wasser gewaschen. Die organische Schicht wird dann noch mit 16 ml einer 10 %igen wässerigen HCl-Lösung und dreimal mit je 10 ml Wasser nachgewaschen. Die organische Schicht wird mit wasserfreiem $Na_2SO_4$ getrocknet und das Lösungsmittel wird im Vakuum abgedampft. Der ausgefallene Niederschlag wird abgefiltert, im Vakuum getrocknet und aus Aceton umkristallisiert. Es werden 4,4 g (85,4%) der Titelverbindung (Nicardipine-Hydrochlorid) erhalten.

Beispiel 2

2,6-Dimethyl-4-(3'-nitrophenyl)-1,4-dihydropyridin-3,5-dicarbonsäure-3β-chloräthylester-5-methylester

Ein Gemisch von 3,32 g (0,01 Mol) 2,6-Dimethyl-4-(3'-nitrophenyl)-1,4-dihydropyridin-3,5-dicarbonsäure-3-methylester, 1,43 g (0,01 Mol) 1-Brom-2-chloräthan und 1,01 g (0,01 Mol) Triäthylamin wird bei 120°C 8 Stunden gerührt. Das Reaktionsgemisch wird dann in einem Aethylacetat/Methanol/Ammoniak-Gemisch (80:30:3) gelöst und an einer Kieselgelsäule ( 2 x 35 cm) chromatographiert. Es werden 0,75 g (19,8 %) 2,6-Dimethyl-4-(3'-nitrophenyl)-1,4-dihydropyridin-3,5-dicarbonsäure-3β-chloräthylester-5-methylester und 0,1 g (1,5 %) des dimeren 1,2-Bis/methyl-2,6-dimethyl-4-(3'-nitrophenyl)-1,4-dihydropyridin-3,5-dicarboxy/äthans als ölige Substanzen erhalten.

Bruttoformel: $C_{18}H_{19}ClN_2O_6$
Molekulargewicht: 394
NMR ($CDCl_3$) der Titelverbindung:

δ : 2,4 (6H, s, $C_{2,6}-CH_3$)　　3,65 (3H, s, $-COOCH_3$)
3,65 (2H, t, $-CH_2Cl$)　　4,3 (2H, t, $-COOCH_2-$)
5,15 (1H, s, $C_4-H$)　　6,3 (1H, s, >N-H)

NMR ($CDCl_3$) der dimeren Verbindung:

δ : 1,8 (6H, s, $C_{2,6}-CH_3$)　　2,4 (3H, s, $-COOCH_3$)
3,6 (2H, s, $-COOCH_2$)　　4,6 (1H, s, $C_4-H$)
5,6 (1H, s, >N-H)

Die Titelverbindung kann auf die im Beispiel 7 der DE-PS 24 07 115 beschriebene Weise zu Nicardipine bzw. dessen Hydrochlorid umgesetzt werden.

## Patentansprüche

1. Verfahren zur Herstellung von 2,6-Dimethyl-4-(3'-nitrophenyl)-1,4-dihydropyridin-3,5-dicarbonsäure-3β-(N-benzyl-N-methylamino)-äthylester-5-methylester der Formel

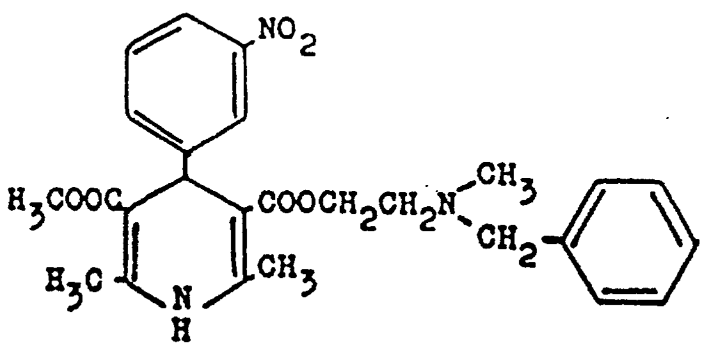

und dessen Hydrochlorid-Salz, dadurch gekennzeichnet, dass man 2,6-Dimethyl-4-(3'-nitrophenyl)-1,4-dihydropyridin-3,5-dicarbonsäure-dimethylester in Anwesenheit von inerten organischen Lösungsmitteln bei einer Temperatur zwischen Raumtemperatur und Rückflusstemperatur des Reaktionsgemisches mit einer wässerigen Alkalihydroxyd-Lösung partiell hydrolysiert und das erhaltene 2,6-Dimethyl-4-(3'-nitrophenyl)-1,4-dihydropyridin-3,5-dicarbonsäure-3-methylester der Formel

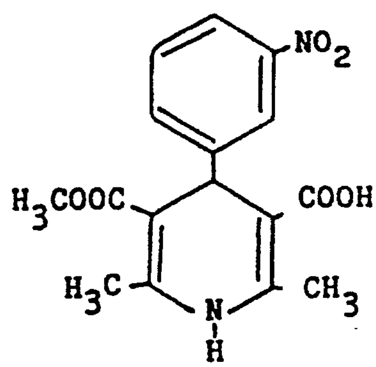

a) entweder mit N-(2-Hydroxyäthyl)-N-benzyl-methylamin der Formel

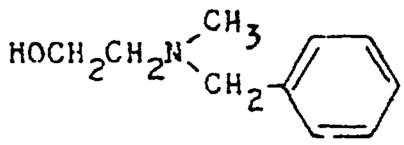

in Anwesenheit von N,N'-Dicyclohexylcarbodiimid bei einer Temperatur zwischen 25°C und 120°C oder mit

b) N-(2-Halogenäthyl)-N-benzyl-methylamin der Formel

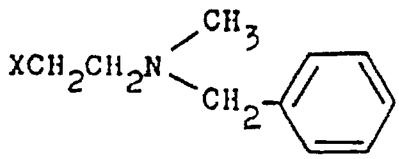

worin X Halogen, wie Chlor oder Brom bedeutet, in Anwesenheit von inerten organischen Lösungsmitteln und eines Protonenakzeptors bei einer Temperatur zwischen 25°C und 140°C zur Titelverbindung umsetzt und diese gewünschtenfalls in ihr pharmazeutisch annehmbares Hydrochlorid-Salz überführt.

2. Verfahren nach Anspruch 1, dadurch gekennzeichnet, dass man die partielle Hydrolyse in Anwesenheit einer wässerigen Natriumhydroxyd-Lösung durchführt.

3. Verfahren nach Anspruch 1, dadurch gekennzeichnet, dass man bei der partiellen Hydrolyse als inertes organisches Lösungsmittel einen niederen Alkohol, wie Methanol, verwendet.

4.Verfahren nach Anspruch 1b), dadurch gekennzeichnet, dass man als inertes organisches Lösungsmittel einen niederen Alkohol, wie n-Butanol, verwendet.

5. Verfahren nach Anspruch 1b), dadurch gekennzeichnet, dass man als Protonenakzeptor eine organische Base, wie Triäthylamin, verwendet.

## Claims

1. A process for the manufacture of 2,6-dimethyl-4-(3'-nitrophenyl)-1,4-dihydropyridine-3,5-dicarboxylic acid 3β-(N-benzyl-N-methylamino)-ethyl ester-5-methyl ester of the formula

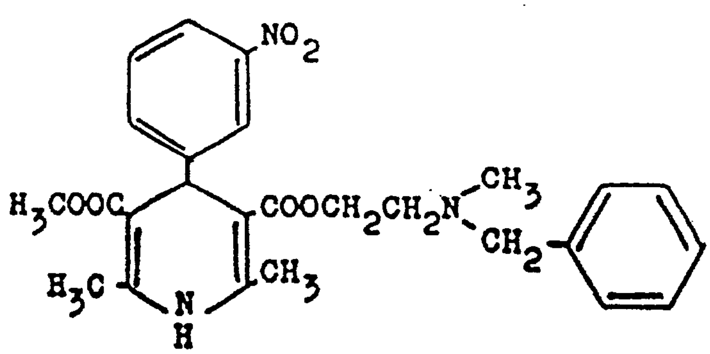

and of its hydrochloride salt, characterized in that 2,6-dimethyl-4-(3'-nitrophenyl)-1,4-dihydropyridine-3,5-dicarboxylic acid dimethyl ester is partially hydroylzed with an aqueous alkali hydroxyde solution in the presence of inert organic solvents at a temperature between room temperature and the reflux temperature of the reaction mixture and the resulting 2,6-dimethyl-4-(3'-nitrophenyl)-1,4-dihydropyridine-3,5-dicarboxylic acid 3-methyl ester of the formula

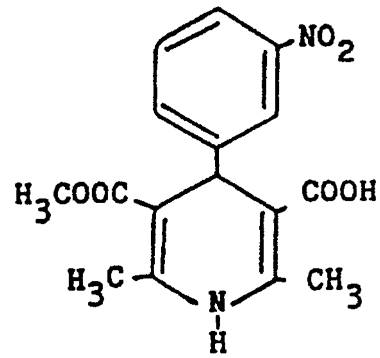

is reacted with

a) either N-(2-hydroxyethyl)-N-benzyl-methylamine of the formula

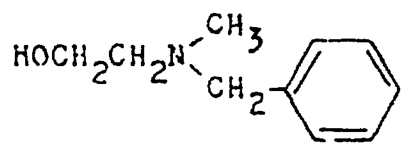

in the presence of N,N'-dicyclohexylcarbodiimide at a temperature between 25°C and 120°C,

b) or N-(2-haloethyl)-N-benzyl-methylamine of the formula

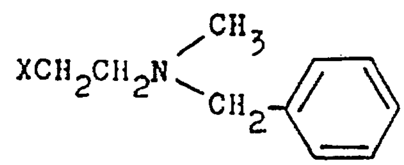

wherein X represents halo such as chloro or bromo in the presence of inert organic solvents and of a proton acceptor at a temperature between 25°C and 140°C, to the title compound, which is optionally converted to its pharmaceutically acceptable hydrochloride salt.

2. A process according to claim 1, characterized in that the partial hydrolysis is effected in the presence of an aqueous solution of sodium hydroxyde.

3. A process according to claim 1, characterized in that a lower alcohol such as methanol is used as the inert organic solvent in the partial hydrolysis.

4. A process according to claim 1b), characterized in that that a lower alcohol such as n-butanol is used as the inert organic solvent.

5. A process according to claim 1b), characterized in that an organic base such as triethylamine is used as the proton acceptor.

**Revendications**

1. Procédé de préparation de l'ester 3-bêta-(N-benzyl-N-méthylamino)-éthyl 5-méthylique d'acide 2,6-diméthyl-4-(3'-nitrophényl)-1,4-dihydropyridine-3,5-dicarboxylique répondant à la formule

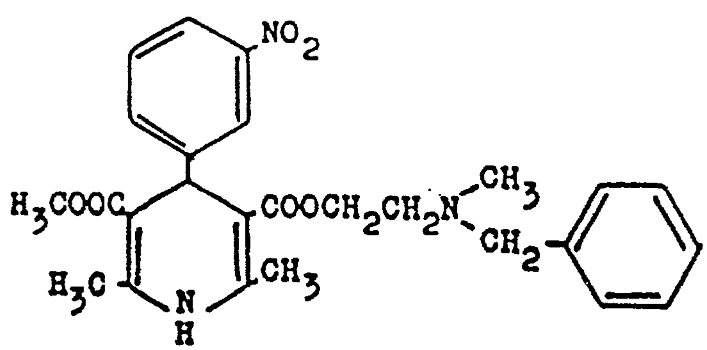

et de son sel hydrochlorique, caractérisé en ce que l'on hydrolyse partiellement un ester diméthylique d'acide 2,6-diméthyl-4-(3'-nitrophényl)-1,4-dihydropyridine-3,5-dicarboxylique au moyen d'une solution aqueuse d'hydroxyde alcalin, en présence de solvants organiques inertes, à une température comprise entre la température ambiante et la température de reflux du mélange réactionnel, et on fait réagir l'ester 3-méthylique d'acide 2,6-diméthyl-4-(3'-nitrophényl)-1,4-dihydropyridine-3,5-dicarboxylique obtenu et répondant à la formule

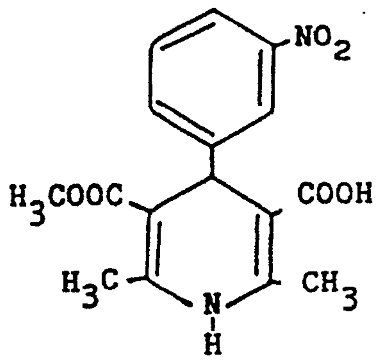

a) soit avec une N-(2-hydroxyéthyl)-N-benzyl-méthylamine de formule

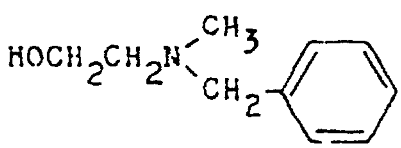

en présence d'un N,N'-dicyclohexylcarbodiimide, à une température comprise entre 25 et 120°C,
b) soit avec une N-(2-halogéno-éthyl)-N-benzyl-méthylamine de formule

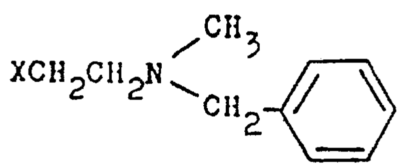

dans laquelle X est un halogène, tel que du chlore ou du brome, en présence de solvants organiques inertes et d'un accepteur de protons, à une température comprise entre 25 et 140°C, pour le transformer en composé titré et, le cas échéant, on le transforme en sel hydrochlorique pharmaceutiquement acceptable.

2. Procédé selon la revendication 1, caractérisé en ce que l'on réalise l'hydrolyse partielle en présence d'une solution aqueuse d'hydroxyde de sodium.

3. Procédé selon la revendication 1, caractérisé en ce que l'on utilise un alcool inférieur tel que du méthanol en tant que solvant organique inerte pour l'hydrolyse partielle.

4. Procédé selon la revendication 1b), caractérisé en ce que l'on utilise un alcool inférieur tel que du n-butanol en tant que solvant organique inerte.

5. Procédé selon la revendication 1b), caractérisé en ce que l'on utilise une base organique telle qu'une triéthylamine en tant qu'accepteur de protons.